**Europäisches Patentamt**

**European Patent Office**

**Office européen des brevets**

(11) Veröffentlichungsnummer: **0 212 094 B1**

# EUROPÄISCHE PATENTSCHRIFT

(45) Veröffentlichungstag der Patentschrift:
**17.11.88**

(21) Anmeldenummer: **86107467.2**

(22) Anmeldetag: **02.06.86**

(51) Int. Cl.⁴: **C 07 D 307/89,** C 07 D 307/60, C 10 L 1/02, C 10 J 3/00, F 23 G 5/033

(54) **Verfahren zur Verwertung von Phthalsäureanhydrid- und/oder Maleinsäureanhydrid-Destillationsrückständen.**

(30) Priorität: **12.07.85 DE 3524919**

(43) Veröffentlichungstag der Anmeldung:
**04.03.87 Patentblatt 87/10**

(45) Bekanntmachung des Hinweises auf die Patenterteilung:
**17.11.88 Patentblatt 88/46**

(84) Benannte Vertragsstaaten:
**AT BE DE FR GB IT**

(56) Entgegenhaltungen:
**AT - B - 261 790**
**DE - B - 1 926 919**
**GB - A - 2 034 349**

**ULLMANNS ENCYKLOPÄDIE DER TECHNISCHEN CHEMIE, 4. Auflage, Band 16, 1978, VERLAG CHEMIE GMBH, Weinheim, New York, pp. 408-411**
**ULLMANNS ENCYKLOPÄDIE DER TECHNISCHEN CHEMIE, 4. Auflage, Band 18, 1979, VERLAG CHEMIE GMBH, Weinheim, Deerfield Beach, Florida, Basel, pp. 522-532**

(73) Patentinhaber: **HÜLS AKTIENGESELLSCHAFT, Patentabteilung / PB 15 - Postfach 13 20, D-4370 Marl 1 (DE)**

(72) Erfinder: **Scharf, Helmut, Dr., Kilianstrasse 75, D-4235 Schermbeck (DE)**
Erfinder: **Krix, Wilfried, Randebrockstrasse 3, D-4250 Bottrop (DE)**

ACTORUM AG

## Beschreibung

Phthalsäureanhydrid (PSA) wird industriell durch Oxidation von o-Xylol und/oder Naphthalin mit Luft in der Gasphase hergestellt. Das bei diesem Prozess anfallende PSA muss, um den hohen Reinheitsforderungen zu genügen, destilliert werden. Hierbei fällt ein Rückstand an, der bei Temperaturen oberhalb von ca. 180°C flüssig ist und beim Erkalten je nach dem Einsatzstoff und den gewählten Destillationsbedingungen eine zähe bis glasartige Masse ergibt. Für die Beseitigung dieses Rückstandes werden in der Technik zwei Verfahren angewandt. Bei dem einen Verfahren wird die Destillation nur soweit betrieben, dass ca. 50% PSA im Rückstand verbleiben. Dieser Rückstand ist bei höheren Temperaturen so niedrig viskos, dass er pumpbar ist. Er wird zu den entsprechenden Verbrennungsanlagen gefördert und dort verbrannt. Diese Beseitigungsmethode hat den Nachteil, dass wertvolles Produkt verbrannt werden muss, nur um den Rückstand in einem förderbaren Zustand zu erhalten.

Bei dem anderen Verfahren wird das PSA soweit ausdestilliert, dass der Rückstand noch eben aus der Destillationsblase abgedrückt werden kann. Je nach dem Destillationsgrad entweicht hierbei PSA, das zu Kontamination der Anlage führen kann. Der in einer Wanne erkaltete Rückstand muss gebrochen und zur Verbrennungsanlage transportiert werden. Diese Handhabung ist umständlich und teuer.

Auch bei der Maleinsäureanhydrid (MSA)-Herstellung fällt ein Destillationsrückstand an. Dieser Rückstand wurde bisher entweder in Wasser gelöst und ins Abwasser gegeben, was eine erhebliche Belastung der Umwelt bedeutet, oder verbrannt (vgl. Ullmanns Encyklopädie der technischen Chemie, 4. Auflage, Band 16, 178; Verlag Chemie GmbH Seite 411 Kapitel 2.2.5).

Es war daher Aufgabe der Erfindung, ein Verfahren zu entwickeln, das die Handhabung der Destillationsrückstände einfacher und sicherer macht, die Umwelt nicht belastet und die Rückstände einer Wertschöpfung zuführt.

Diese Aufgabe wird erfindungsgemäss dadurch gelöst, dass der jeweilige heisse, flüssige Destillationsrückstand als Strahl in eine gekühlte, organische Flüssigkeit fliesst, in der ein Messer mit hohen Umdrehungen bewegt wird. Während des Abkühlens und Erstarrens wird der Rückstand von dem Messer zerschlagen, so dass sich am Boden des Gefässes ein Granulat ansammelt, das zusammen mit der organischen Flüssigkeit zu einer leicht pumpbaren Suspension vermahlen wird, die zur Wasserdampf-Herstellung oder Synthesegas-Erzeugung dient.

Das erfindungsgemässe Verfahren hat den Vorteil, dass der bei der PSA-Destillation anfallende Rückstand in eine auch bei Raumtemperatur leicht handhabbare Form übergeführt wird, so dass die Weiterverarbeitung wesentlich vereinfacht wird. Da bei der PSA-Rückstandsaufarbeitung das Verfahren bevorzugt bei stark ausdestillierten PSA-Rückständen angewendet wird, werden zusätzlich erhebliche Mengen an PSA gewonnen, so dass die Kosten für die PSA-Destillation gesenkt werden. Bei der MSA-Herstellung wird der Anfall von Abwasser vermieden, so dass die Umwelt nicht belastet wird. Darüber hinaus können die Rückstände nicht nur, gegebenenfalls in Kombination mit einer Wasserdampf-Erzeugung, verbrannt werden, sondern sie können, je nach den verwendeten Suspensionsmitteln, auch in Synthesegas umgewandelt werden, so dass eine zusätzliche Wertschöpfung erzielt wird.

Der aus der Destillationsblase kommende PSA-Rückstand ist in seiner chemischen Struktur nicht genau bekannt und kann in seinen Eigenschaften sehr unterschiedlich sein. Je nach Temperatur, die in der Regel zwischen 200 und 250°C liegt, dem Destillationsgrad und vor allem der Ausgangssubstanz, o-Xylol und/oder Naphthalin, kann er dünnflüssig bis hochviskos sein.

Auch beim MSA-Rückstand ist die chemische Zusammensetzung nicht genau bekannt, und das äussere Erscheinungsbild hängt auch bei ihm vom Ausgangsmaterial der MSA-Herstellung (Benzol/$C_4$-Kohlenwasserstoffe) und dem Destillationsgrad ab. Je nach der Temperatur, die in der Regel zwischen 170 und 180°C liegt, und dem Gehalt an Fumarsäure ist er dünnflüssig bis breiig.

Als Suspensionsmittel kommen bei Raumtemperatur flüssige organische Substanzen infrage. Da sie in der Regel zusammen mit dem PSA- und MSA-Rückstand verbrannt oder zu Synthesegas umgewandelt werden, sollten sie kein oder nur sehr wenig Halogen, Schwefel und Stickstoff enthalten. Sauerstoffhaltige Verbindungen sind dagegen geeignet. Bevorzugt werden aliphatische und/oder aromatische Kohlenwasserstoffe, wie z.B. Paraffinöl und leichtes Heizöl. Besonders bevorzugt werden flüssige organische Abfälle aus anderen Produktionsprozessen, wie z.B. die Destillationsrückstände aus Alkylierungsanlagen (z.B. die Rückstände von Ethylbenzol und dessen Folgeprodukte Styrol, Cumol sowie längerkettige Paraffine, gegebenenfalls im Gemisch mit Aromaten aus der Tensid-Herstellung) und aus der Alkohol-Herstellung (wie z.B. die Destillationsrückstände von n- und iso-Butanol sowie 2-Ethylhexanol). Wegen der Vielzahl der möglichen Suspensionsmittel muss im Einzelfall experimentell geprüft werden, ob die Verbindung für die Granulierung und Suspendierung des PSA- und MSA-Rückstandes geeignet ist.

Die Abkühlung und Granulierung des PSA- und MSA-Rückstandes hängt von der Beschaffenheit der Rückstände und von dem Suspendiermittel ab. Da sich die Rückstände in ihren äusseren Eigenschaften stark ändern können, und für die Suspendiermittel eine grössere Auswahl besteht, muss im Einzelfall experimentell geprüft werden, ob die Granulierung für die anschliessende Suspendierung ausreicht. Die folgenden Angaben können daher nur als Richtwerte dienen. Da sich die Aufarbeitungen nach dem erfindungsgemässen Verfahren für die PSA- und MSA-Rückstände nicht wesentlich unterscheiden, wird das Verfahren im folgenden der Einfachheit halber nur für den PSA-Rückstand näher beschrieben.

Der PSA-Rückstand fliesst im freien Fall in das Suspensionsmittel, wobei der Durchmesser des Strahls je nach dem gewünschten Durchsatz und den äusseren Eigenschaften des PSA-Rückstandes zwischen ca. 1 und ca. 10 cm schwanken kann. In der Regel beträgt der Durchmesser 2 - 5 cm. Die Länge der Abkühlstrecke, das heisst die Höhe der Flüssigkeitssäule, muss so ausgelegt werden, dass das nach dem Passieren dieser Kühlstrecke vorliegende Granulat soweit erstarrt ist, dass es in Gegenwart des Kühlmittels, das in der Regel auch das Suspendiermittel ist, gemahlen werden kann. Die Länge der Kühlstrecke hängt ausser von den Eigenschaften und der Menge des PSA-Rückstandsstrahles vor allem von der Temperatur des Kühlmittels und der Umdrehungszahl des in dem Kühlmittel rotierenden Messers ab. Bei sehr feinem Strahl (0,5 cm) reicht z.B. eine Flüssigkeitssäule von 30 cm. Bei den in der Technik üblichen Durchmessern von 2 - 5 cm beträgt die Höhe der Flüssigkeitssäule ca. 80 - 120 cm. Massgebend für die Menge an Kühlflüssigkeit ist die nach der Abschreckung des PSA-Rückstandes sich einstellende Mischtemperatur. Die Begrenzung der Mischtemperatur nach unten ist gegeben durch die Zunahme der Viskosität des Kühlmittels mit fallender Temperatur. Da für die Weiterverarbeitung des Granulats möglichst niedrige Viskositäten angestrebt werden, und die Handhabung der Mischung bei Raumtemperatur am einfachsten ist und zudem eine Kühlung unter die Raumtemperatur sehr teuer ist, wird als untere Temperaturgrenze die Umgebungstemperatur bevorzugt. Die obere Grenze der Mischtemperatur hängt von den Eigenschaften des PSA-Rückstandes, vor allem aber von der Art des Kühlmittels, ab. Sie sollte nach Möglichkeit so gewählt werden, dass das Kühlmittel den PSA-Rückstand nicht oder nur sehr wenig anlöst. In der Regel liegt diese Temperatur bei ca. 60 - 90°C. Bei noch höheren Temperaturen kommen die spezifischen Löseeigenschaften der Kühlmittel immer mehr zum Tragen. So kann z.B. PSA-Rückstand mit einem Gemisch aus Styrol-, Ethylbenzol- und Cumol-Destillationsrückstand bei 120 - 130°C in eine Lösung/Emulsion überführt werden, die bei diesen Temperaturen pumpbar ist, die jedoch beim Abkühlen zu einer teerartigen und kaum noch zu handhabenden Masse erstarrt.

Verfahrenstechnisch hängt die Menge an Kühlflüssigkeit davon ab, ob die PSA-Destillation kontinuierlich oder diskontinuierlich betrieben wird, welche Mengen PSA-Rückstand pro Zeiteinheit granuliert werden sollen, welche Temperatur der PSA-Rückstand und welche Temperatur das Kühlmittel besitzt und ob das Kühlmittel über eine externe Kühlvorrichtung im Kreis gefahren wird.

Das in der Flüssigkeit rotierende Messer hat zwei Funktionen. Zum einen soll es den erstarrenden PSA-Strahl zerhacken, zum anderen muss es die Kühlflüssigkeit in so starke Rotation versetzen, dass ein guter Wärmeübergang für die Abschreckung des PSA-Rückstandsstrahles gegeben ist. Andererseits darf die Fläche des Messers senkrecht zur Drehrichtung nicht zu gross sein, da sich sonst an der Oberfläche noch nicht voll erstarrter PSA-Rückstand ansetzen und dort erstarren kann, was zu einer Verklumpung des PSA-Rückstandes führen würde. Im einfachsten Fall besteht das Messer aus einem schmalen Flacheisen. Es kann jedoch auch ein mehrflügeliger Propeller sein. Da das Flacheisen die gestellten Forderungen am einfachsten erfüllt, wird es in der Regel bevorzugt. Die Justierung des Messers in der Flüssigkeitssäule hängt von der Dicke, der Sinkgeschwindigkeit und der Erstarrungszeit des PSA-Rückstandsstrahles ab. In der Regel beträgt die Höhe der Flüssigkeitssäule über dem Messer ca. 10 - 50 cm. Die Umdrehungszahl des Messers kann in einem sehr weiten Bereich schwanken. Auch sie hängt von den Eigenschaften des PSA-Rückstandsstrahles und den Eigenschaften, vor allem der Viskosität, des Kühlmittels ab. Darüber hinaus richtet sich die Umdrehungszahl nach der gewünschten Korngrösse des Granulats. Je höher die Umdrehungszahl ist, um so feiner fällt das Granulat ein. So wird z.B. in paraffinischen Kohlenwasserstoffen oder in leichtem Heizöl bei 600 Umdrehungen in der Minute ein Kornspektrum von ca. 5 - 25 mm erhalten. In den gleichen Kühlmitteln beträgt die Korngrösse bei 1000 Umdrehungen pro Minute nur noch ca. 1 mm (paraff. Kohlenwasserstoffe) und 1 - 3 mm (leichtes Heizöl). Da für die Weiterverarbeitung ein feines Korn günstiger ist, werden in der Regel Umdrehungszahlen von ca. 1000 bis 3000 Umdrehungen pro Minute bevorzugt.

Die Bereitstellung der Kühlenergie zur Abkühlung des PSA-Rückstandes erfolgt durch Umpumpen der Kühlflüssigkeit über eine Kühlstrecke, die innerhalb oder ausserhalb des Granuliergefässes angeordnet sein kann. Da bei dem anschliessenden Mahlschritt noch zusätzlich Wärme erzeugt wird, wird eine externe Kühlung in Kombination mit der Mahlung bevorzugt. Das Verhältnis von PSA-Rückstand zu umgepumptem Kühlmittel muss so eingestellt werden, dass die maximal zulässige Mischtemperatur, die in der Regel ca. 60 - 90°C beträgt, nicht überschritten wird. So werden z.B. bei Annahme, dass die Wärmekapazität des Kühlmittels der des PSA-Rückstandes entspricht, zur Abschreckung von 1 t PSA-Rückstand pro Stunde von 220°C auf 60°C, 8 t Kühlmittel pro Stunde benötigt, wenn die Kühlstrecke so ausgelegt wird, dass das Kühlmittel beim Umpumpen von 60°C auf 40°C abgekühlt wird.

Der bei der erfindungsgemässen Granulierung anfallende PSA-Rückstand kann von dem Kühlmittel getrennt und separat verbrannt werden oder erfindungsgemäss zusammen mit dem Kühlmittel zu einer pumpbaren Suspension weiterverarbeitet werden. Es ist jedoch auch möglich, die Kühlung und Suspendierung mit unterschiedlichen Flüssigkeiten durchzuführen. Dies kann z.B. aus ökonomischen Gründen sinnvoll sein, wenn der Ort des Anfallens des Rückstandes von dem Ort der Weiterverarbeitung räumlich weit entfernt ist, oder es kann sogar geboten sein, wenn der Transport des Suspendiermittels aus ökologischen Gründen unerwünscht ist. Die erfindungsgemässe Umarbeitung des Granulats in eine Suspension wird in der Regel mit in der Technik bekannten Hilfsmitteln, wie z.B. Kolloidmühlen durchgeführt. Die Feinmahlung des Granulats in Gegenwart des Kühlmittels, das erfindungsgemäss gleichzeitig als Suspendiermittel dient, kann in ein oder zwei Schritten und im geraden Durchgang oder in Kreisfahrweise erfolgen, je nach der Körnung des Granulats, der gewünschten Endfeinheit des Korns in der Suspension und der durchzusetzenden Menge.

Die Feinheit des Korns in der Suspension richtet sich nach der gewünschten Beständigkeit der Suspension. Wenn die Suspension sofort weiterverarbeitet oder permanent gerührt wird, dann braucht das Korn nicht so fein zu sein, als wenn die Suspension für einige Zeit lagerstabil sein muss. Darüber hinaus hängt die einzustellende Mahlfeinheit auch von der Art des Suspendiermittels ab. So reicht z.B. bei Verwendung eines Destillationsrückstandes aus der Styrol-, Ethylbenzol- und Cumol-Herstellung eine Kornfeinheit von 20 - 150 μm aus, um eine für einige Tage stabile Suspension zu erhalten, während für die Suspendierung in leichtem Heizöl die Korngrösse im Bereich von ca. 10 - 50 μm liegen sollte. Bevorzugt werden in der Regel Korngrössen < 50 μm.

Das Verhältnis PSA-Rückstand zu Suspendiermittel richtet sich hauptsächlich nach den apparativen Möglichkeiten zur Weiterverarbeitung der Suspension. Aus wirtschaftlichen Gründen wird ein möglichst hoher Gehalt an PSA-Rückstand in der Suspension angestrebt. Mit zunehmendem Gehalt an PSA-Rückstand nimmt die Viskosität der Suspension stark zu, so dass z.B. bei Einspeisung der Suspension in eine Synthesegas-Anlage Schwierigkeiten bei den Hochdruckpumpen und den Ventilen entstehen. In der Regel beträgt das Verhältnis PSA-Rückstand zu Suspendiermittel 1-2 : 1. Die Viskosität beträgt hierbei je nach dem Suspendiermittel bis zu ca. 1500 mPa s.

Die Feinmahlung und die Einstellung des Verhältnisses kann in einem Schritt geschehen, indem z.B. das Granulat aus dem Granulator mit Hilfe einer Förderschnecke in die Kolloidmühle gefördert wird. Die zwischen dem Granulat sich befindende Flüssigkeit reicht häufig aus, ein Verhältnis von ca. 1 : 1 einzustellen. Bei sehr grobem Granulat ist es angebracht, den Mahlschritt in 2 Stufen durchzuführen, um den Durchsatz an PSA-Rückstand zu erhöhen, indem in der ersten Mahlstufe z.B. das Korn von 3 mm auf 0,5 mm und in der 2. Stufe von 0,5 mm auf < 50 μm gemahlen wird. Bei diesem geraden Durchgang des PSA-Rückstandes durch die Granulierung und die Grob- und Feinmahlung ist es erforderlich, dass bei der Granulierung das Kühlmittel über eine externe Kühlung im Kreis gefahren wird oder aber das Kühlaggregat in dem Granuliergefäss sich befindet und die Kühlflüssigkeit kräftig umgewälzt wird. Eine andere und bevorzugte Ausführung des erfindungsgemässen Verfahrens besteht in der Kombination der Kühlung bei der Granulierung mit der Grobmahlung. Hierbei wird das aus der Grobmahlung kommende Gemisch über eine Kühlung in das Granuliergefäss zurückgeführt, wobei die Mühle gleichzeitig als Förderpumpe dient. In einem Seitenstrom wird Produkt aus der Grobmahlung über eine Feinmühle abgezogen und zu einem Vorratsbehälter oder zur Synthesegas-Anlage gepumpt. In der Regel wird die zu bearbeitende Menge an PSA-Rückstand vorgegeben, eine gleich grosse Menge an frischem Kühlmittel in das Granuliergefäss zugegeben und das Produkt aus der Grobmahlung zum grössten Teil im Kreis gefahren, während ein kleinerer Teil, der mengemässig der Menge aus PSA-Rückstand und aus frischem Kühlmittel entspricht, über die Feinmühle als fertige Suspension (PSA-Rückstand : Suspendiermittel = 1 : 1) zur Weiterverarbeitung abgezweigt wird.

Bei einer anderen Ausführungsform des erfindungsgemässen Verfahrens wird die fertige Suspension in den Vorratsbehälter für das Kühlmittel gepumpt und zusammen mit dem Kühlmittel (= Suspendiermittel) zur Granulierung geführt.

Das beschriebene Verfahren ist erfindungsgemäss auch auf die Aufarbeitung des Destillationsrückstandes des Maleinsäureanhydrids anwendbar. Darüber hinaus ist es allgemein auf solche organischen Stoffe übertragbar, die bei höheren Temperaturen flüssig vorliegen und die bei Raumtemperatur fest und mahlbar sind.

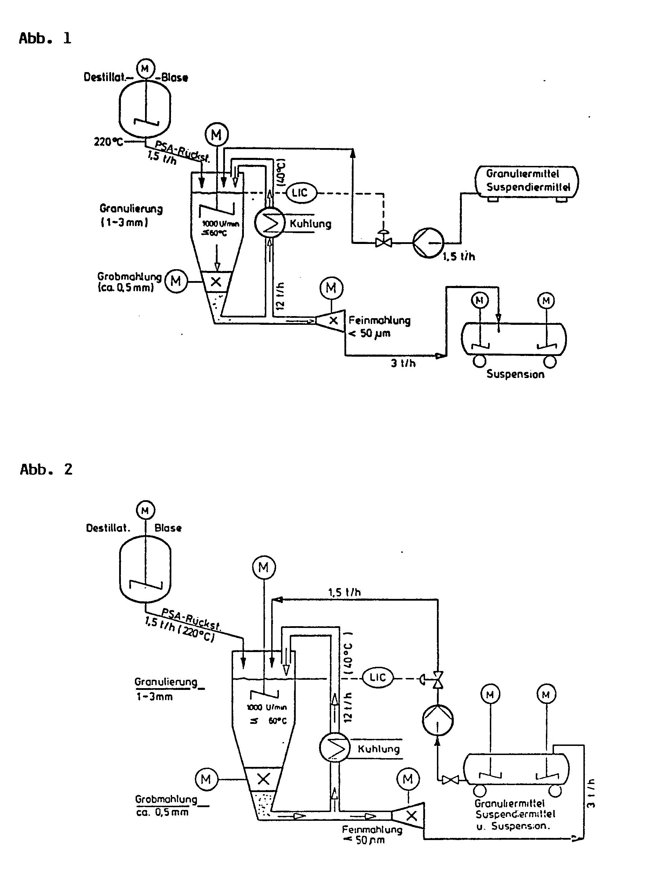

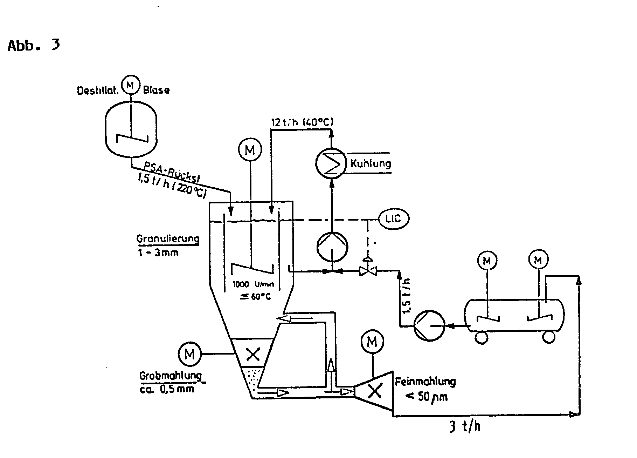

In den Abbildungen 1 - 3 sind einige Ausführungsbeispiele des erfindungsgemässen Verfahrens wiedergegeben.

Abb. 1 betrifft die Suspendierung von PSA-Destillations-Rückstand mit Integrierter Granulierung und Grobmahlung.

Abb. 2 betrifft die Suspendierung von PSA-Destillations-Rückstand mit integrierter Granulierung, Grobmahlung und Feinmahlung.

Abb. 3 betrifft die Suspendierung von PSA-Destillations-Rückstand mit integrierter Granulierung und Feinmahlung.

## Patentansprüche

1. Verfahren zur Verwertung von Phthalsäureanhydrid- und/oder Maleinsäureanhydrid-Destillationsrückständen, dadurch gekennzeichnet, dass

   a) die heissen und flüssigen Destillationsrückstände als Strahl in einer organischen Flüssigkeit abgeschreckt werden,

   b) die in der organischen Flüssigkeit erstarrenden Destillationsrückstände durch ein hochtouriges Messer zerkleinert werden,

   c) das entstehende Granulat, ggf. unter weiterer Mahlung, in einer organischen Flüssigkeit suspendiert wird und

   d) die Suspension verbrannt oder zu Synthesegas verarbeitet wird.

2. Verfahren nach Anspruch 1, dadurch gekennzeichnet, dass für die Abschreckung und Granulierung und für die Suspendierung der Destillationsrückstände verschiedene organische Flüssigkeiten verwendet werden.

3. Verfahren nach Anspruch 1, dadurch gekennzeichnet, dass für die Abschreckung und Granulierung und für die Suspendierung der Destillationsrückstände ein und dieselbe organische Flüssigkeit verwendet wird.

4. Verfahren nach Anspruch 2 und 3, dadurch gekennzeichnet, dass die organischen Flüssigkeiten Destillationsrückstände aus anderen Herstellungsprozessen sind.

5. Verfahren nach Anspruch 2 und 3, dadurch gekennzeichnet, dass als organische Flüssigkeit leichtes Heizöl verwendet wird.

6. Verfahren nach Anspruch 1 bis 5, dadurch gekennzeichnet, dass die Kühlflüssigkeit über ein Kühlaggregat im Kreis gefahren wird.

7. Verfahren nach Anspruch 1 bis 5, dadurch gekennzeichnet, dass ein Teil der Kühlflüssigkeit zusammen mit grobgemahlenem Granulat und/oder einem Teil der fertigen Suspension über ein Kühlaggregat in die Abschreckstufe zurückgeführt wird.

## Claims

1. A process for utilizing distillation residues from phthalic anhydride and/or maleic anhydride, characterized in that

a) the hot liquid distillation residue in the form of a stream is quenched in an organic liquid,

b) the distillation residue solidifying in the organic liquid is comminuted by a knife turning at high speed,

c) the resulting granules, sometimes after further milling, are suspended in an organic liquid and,

d) the suspension is burned or processed to give synthetic gas.

2. A process according to Claim 1, characterized in that different organic liquids are used for quenching and granulation and for suspending the distillation residues.

3. A process according to Claim 1, characterized in that one and the same organic liquid is used for quenching and granulation and for suspending the distillation residues.

4. A process according to Claims 2 and 3, characterized in that the organic liquids are distillation residues from other production processes.

5. A process according to Claims 2 and 3, characterized in that light fuel oil is used as organic liquid.

6. A process according to Claims 1 to 5, characterized in that the cooling liquid is circulated through a cooling apparatus.

7. A process according to Claims 1 to 5, characterized in that a part of the cooling liquid together with coarsely milled granulate and/or part of the final suspension is added back to the quenching stage via the cooling apparatus.

## Revendications

1. Procédé de mise en valeur des résidus de distillation d'anhydre phtalique et/ou d'anhydride maléique, caractérisé en ce que:

a) les résidus de distillation chauds et liquides sont refroidis brusquement dans un liquide organique sous forme de jets,

b) les résidus de distillation qui se solidifient dans le liquide organique sont broyés à l'aide d'un couteau tournant à haute vitesse,

c) le granulé en résultant, le cas échéant sans broyage complémentaire, est mis en suspension dans un liquide organique, et

d) la suspension est calcinée ou transformée en gaz de synthèse.

2. Procédé selon la revendication 1, caractérisé en ce que, pour le refroidissement busque et la granulation et pour la mise en suspension des résidus de distillation, différents liquides organiques sont utilisés.

3. Procédé selon la revendication 1, caractérisé en ce que pour le refroidissement brusque et la granulation et pour la mise en suspension des résidus de distillation, un seul et même liquide organique est utilisé.

4. Procédé selon les revendications 2 et 3, caractérisé en ce que les liquides organiques sont des résidus de distillation à partir d'autres processus de fabrication.

5. Procédé selon les revendications 2 et 3, caractérisé en ce que l'on utilise comme liquide organique du pétrole léger.

6. Procédé selon les revendications 1 à 5, caractérisé en ce que le liquide de refroidissement est amené par l'intermédiaire d'un agrégat de refoidissement circulant en boucle.

7. Procédé selon les revendications 1 à 5, caractérisé en ce qu'une partie du liquide de refroidissement est ramenée ensemble avec un granulé broyé grossièrement et/ou une partie de la suspension achevée, par l'intermédiaire d'un agrégat de refroidissement, dans l'étape de trempe.

**Abb. 1**

**Abb. 2**

**Abb. 3**

Destillat. (M) Blase

PSA-Rückst
1,5 t/h (220°C)

12 t/h (40°C)

Kühlung

LIC

Granulierung
1 - 3mm

1000 U/mn
≦ 60°C

1,5 t/h

Grobmahlung
ca. 0,5 mm

Feinmahlung
< 50 µm

3 t/h